# EUROPEAN PATENT APPLICATION

(11) **EP 0 903 112 A2**
(43) Date of publication of application: **24.03.1999**
(21) Application number: 98114894.3
(22) Date of filing: 07.08.1998
(51) Int. Cl.: A61B 17/17

(54) **Device for detecting the position of the locking screws of an intramedullary nail.**

(30) Priority: 15.09.1997 IT BO970561
(71) Applicant: HIT MEDICA S.r.L., 47900 Rimini (IT)
(72) Inventor: Iaia, Ciro Rosario, 47900 Rimini (IT)
(74) Representative: Modiano, Guido, Dr.-Ing.

(57) **Abstract**

A device for detecting the position of the locking screws of a tubular nail (1) inserted in the medullary canal of a long bone (3), with passage holes (4,5) for the locking screws formed in the nail proximate to the insertion tip (2). The device comprises a stem (8) which can be inserted in the nail (1) and is provided with means which are suitable to provide rotary coupling to the nail (1) proximate to the passage holes. The stem (8) has an end (10) protruding from the nail (1) and whereto an arm (13) can be rotationally coupled. The arm protrudes outside the bone (3) and supports a template (14) which has guiding holes (15,16) for a drilling tool. The template (14) can be arranged on the stem (8) so as to align the guiding holes (15,16) of the template (14) with the passage holes (4,5) of the nail (1) and so as to allow to drill the bone (3) in alignment with the passage holes (4,5) and allow to insert the locking screws.

## Description

The present invention relates to a device for detecting the position of the locking screws of an intramedullary nail.

Intramedullary nails are used to treat fractures of long bones (femur, tibia, humerus, radius and ulna); the nails are inserted in the medullary canal and fixed, at their opposite ends, by means of through screws. While fixing the screws at the nail insertion end entails no particular problems, fixing the screws at the opposite end is often critical, owing to the fact that the nail, during insertion, undergoes torsions and deformations which produce a misalignment of the position of the screw passage holes which cannot be determined from outside.

The aim of the present invention is to provide a device which allows to detect the position of the screw passage holes after the intramedullary nail has been inserted in the bone.

Within the scope of this aim, an object of the present invention is to provide a device which is highly flexible in use, so that it can be used with nails for treating fractures related to pseudarthrosis of the limbs and to perform length-increasing or -reducing and directional osteotomies.

This aim, this object and others which will become more apparent hereinafter, are achieved with a device for detecting the position of the locking screws of a tubular nail inserted in the medullary canal of a long bone, passage holes for said locking screws being formed in said nail proximate to the insertion tip, characterized in that it comprises a stem which can be inserted in the nail and is provided with means which are suitable to provide rotary coupling to said nail proximate to said passage holes, said stem having an end protruding from said nail and whereto an arm can be rotationally coupled, said arm protruding outside said bone and supporting a template which has guiding holes for a drilling tool, said template being arrangeable on said stem so as to align the guiding holes of said template with the passage holes of said nail and so as to allow to drill the bone in alignment with said passage holes and allow to insert the locking screws.

Further characteristics and advantages of the present invention will become apparent and evident from the following description of a preferred embodiment, illustrated only by way of non-limitative example in the accompanying drawings, wherein:

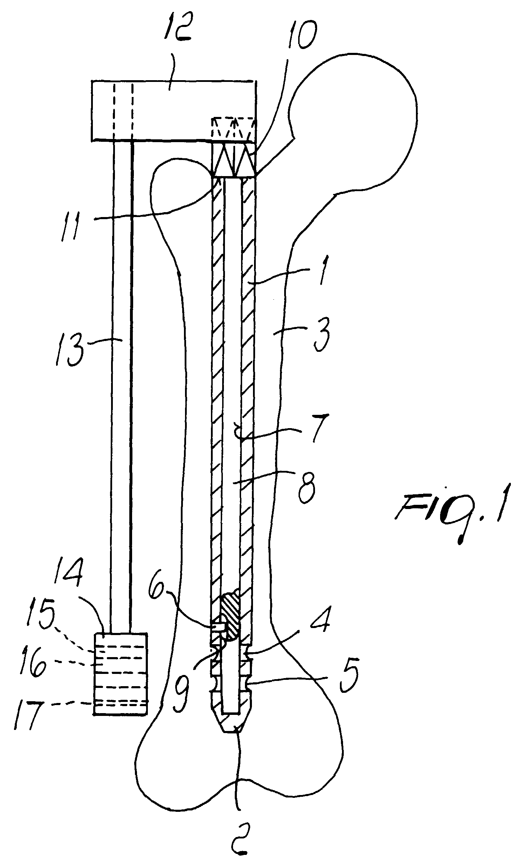

Figure 1 is a view of the device applied to a bone;

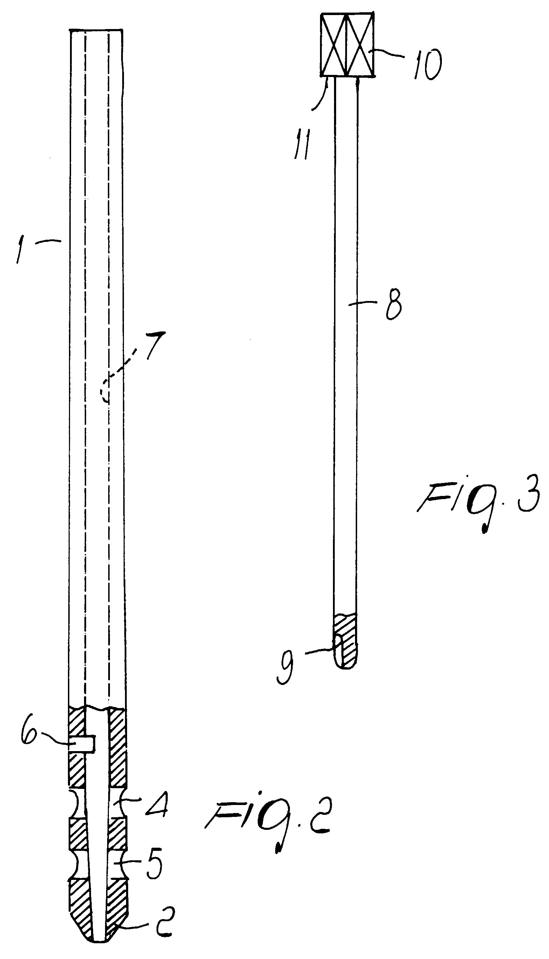

Figure 2 is a view of an intramedullary nail which has been modified to allow to position the device;
Figure 3 is a view of the stem that rotationally couples to the intramedullary nail of Figure 1;

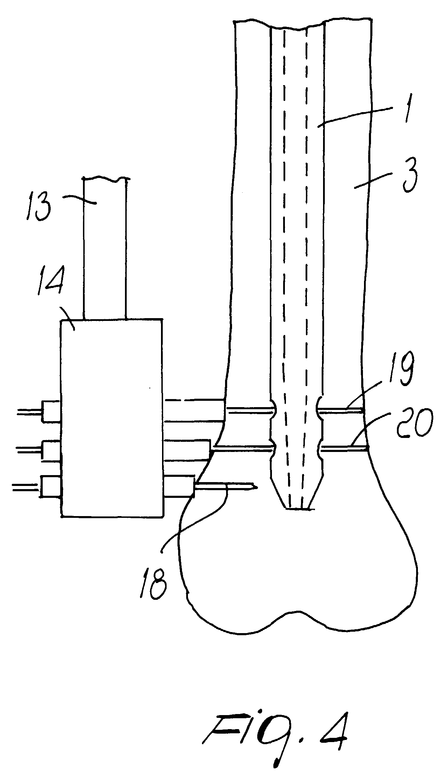

Figure 4 is a view of the template of the device in the position for inserting guiding wires for producing the holes through which the screws for fixing the intramedullary nail are driven; and finally

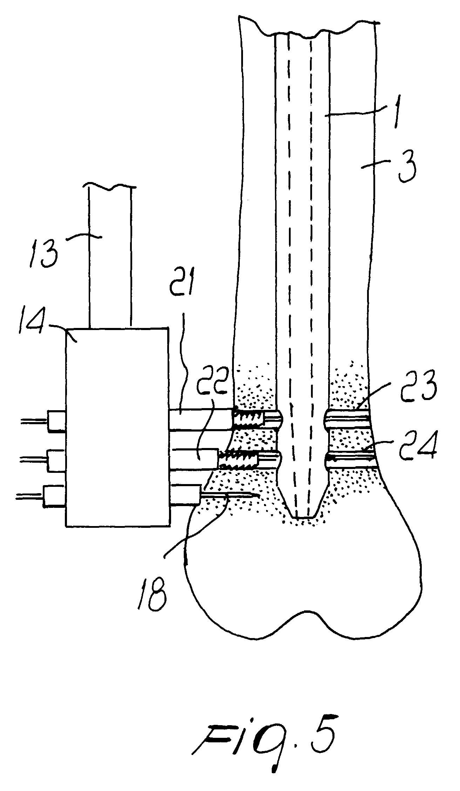

Figure 5 is a view of the template with the drilling tools in the operating position.

With reference to the above figures, 1 designates a tubular intramedullary nail which has a tip 2 by means of which it is inserted in the intramedullary canal of a long bone 3.

At the tip 2, in a conventional manner, two through holes 4 and 5 are provided which lie one above the other on a diametrical plane.

A dowel 6 is fixed through the wall of the nail 1, directly above the hole 4, and protrudes into the internal cavity 7 of the nail 1.

The nail 1 is meant to accommodate a stem 8 whose cross-section is complementary to the internal cross-section of the nail. The stem 8 has a notch 9 at one end and, at the opposite end, a head 10 which is prism-shaped and forms a shoulder 11 together with the stem 8. The notch 9 is open axially and onto the outer face of the stem 8 to allow the dowel 6 to engage in it when the stem 8 is inserted in the nail 1 and thus provide a coupling which rotationally joins the stem 8 to the nail 1.

The length of the stem 8 is such that once the stem has been inserted so that the dowel 6 engages the notch 9, the shoulder 11 of the head 10 rests on the mouth of the internal cavity of the nail.

A block 12 can be rigidly applied to the head 10 and is elongated radially with respect to the stem 8; an arm 13 is fixed to said block and protrudes parallel to the stem 8, at a distance whereat it does not interfere with the bone 3 and with any tissues that cover the bone.

The arm 13 is advantageously constituted by a graduated bar; a template 14 can be arranged at the end of said bar which is opposite with respect to the block 11. In order to allow to adjust its position on the bar 13 with respect to the block 12, the template or the block 12 has screw-type locking means which comprise, for example, a sliding hole in the bar 13; a screw protrudes into the hole and, by acting on the bar 13, locks the template 14 or the block 12 at the chosen level, which can be verified by the graduations on the bar.

In the template 14 two holes 15 and 16 are provided, whose distance one from another is equal to the distance of the holes 4 and 5 of the nail, and a third hole 17, which is co-planar and parallel to the holes 15 and 16.

The template 14 is arranged on the bar 13 so that the holes 15 and 16 are spaced from the shoulder 11 of the head 10 by an extent which is equal to the distance of the holes 4 and 5 from the top of the nail 1.

The coupling of the head 10 to the block 12 is also such as to ensure the orientation of the template 14, so that the holes 15 and 16 are aligned with the respective holes 4 and 5 of the nail 1.

The method of application of the described device is as follows.

First of all, the stem 8 is inserted in the cavity 7 of the intramedullary nail 1, which has already been driven into the medullary canal. By turning the stem 8, one searches for alignment of the notch 9 with the dowel 6; then, once the alignment has been established, an axial action is applied to the stem 8 until the shoulder 11 abuts against the mouth of the nail, so as to axially position the stem inside the nail and rotationally couple the stem and the nail to each other.

Once the stem 8 has been inserted in the nail 1, the block 12 is fixed to the head of the stem 8 with the orientation established by the notch 9 with respect to which the block 12 is fixed. Since the position of the template along the bar 13 has already been set according to the distance between the holes 4, 5 and the mouth of the nail 1, the holes 15, 16 of the template 14 are thus perfectly aligned with the holes 4, 5 of the nail 1.

At this point, after appropriate drilling, an anchoring wire 18 is inserted in the bone; by means of the wire 18, the template 14 is anchored so as to prevent it from moving with respect to the nail. Then the guiding wires 19 and 20 are applied through the holes 4 and 5 and, by using cannulated drill bits 21 and 22, the bone is drilled so as to form holes 23, 24 which are aligned with the holes 4, 5.

After removing the device, the screws which allow to block the end of the nail 1 inside the medullary cavity are driven into the holes 23, 24.

It is evident that the device perfectly achieves the intended aim. In particular, it should be observed that the holes 15, 16 of the template 14 stay aligned with the holes 4, 5 of the nail at all times, even when said nail, during insertion in the medullary canal, is subjected to torsions which produce an angular offset of the end 2 with respect to the mouth of the nail. The position of the dowel 6 proximate to the holes 4 and 5 in fact allows to bypass the portion of the nail in which torsional and flexural deformations mainly occur, preventing them from acting on the device and causing misalignment of the template 14 with respect to the holes 4 and 5.

The disclosures in Italian Patent Application No. BO97A000561 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A device for detecting the position of the locking screws of a tubular nail (1) inserted in the medullary canal of a long bone (3), passage holes (4, 5) for said locking screws being formed in said nail proximate to the insertion tip (2), characterized in that it comprises a stem (8) which can be inserted in the nail (1) and is provided with means (6, 9) which are suitable to provide rotary coupling to said nail (1) proximate to said passage holes (4, 5), said stem (8) having an end (10) which protrudes from said nail and whereto an arm (13) can be rotationally coupled, said arm protruding outside said bone (3) and supporting a template (14) which has guiding holes (15, 16) for a drilling tool, said template (14) being arrangeable on said stem (8) so as to align the guiding holes (15, 16) of said template (14) with the passage holes (4, 5) of said nail (1) and so as to allow to drill the bone in alignment with said passage holes (4, 5) and allow to insert the locking screws.

2. A device according to claim 1, characterized in that said rotary coupling means are constituted by a dowel (6) which protrudes inside said nail (1) proximate to said holes (4, 5) and a notch (9) which is formed at the end of said stem (8) and can be engaged by said dowel (6) when said stem (8) is inserted in said nail (1).

3. A device according to claim 2, characterized in that said stem (8) has, at the end that lies opposite to said notch (9), a head (10) which is prism-shaped and which forms a shoulder (11) with the stem (8), said notch (9) being open axially and on the outer face of said stem (8) to allow said dowel (6) to engage in it when said stem (8) is inserted in said nail (1) to provide a rotational coupling, the length of said stem (8) being such that once said stem has been inserted until said rotary coupling is produced, said shoulder (11) rests on the mouth of the internal cavity (7) of said nail (1).

4. A device according to claim 3, characterized in that a coupling block (12) for said arm (13) can be rigidly fixed to said head (10), said arm (13) being constituted by a graduated bar, a template (14) being arrangeable at the opposite end of said bar with respect to said block (12), means being provided to adjust the position of said template (14) with respect to said block (12) so that the distance of the holes (4, 5) of said nail (1) from said shoulder (11) of the head (10) is equal to the distance of said holes (4, 5) from the mouth of said nail, the fixing of said head (10) to said block (12) being such as to ensure the orientation of said template (14) so that the guiding holes (15, 16) are aligned with the respective holes (4, 5) of the nail.
